# EUROPEAN PATENT APPLICATION

(11) **EP 4 252 858 A1**
(43) Date of publication of application: **04.10.2023**
(21) Application number: 21897979.7
(22) Date of filing: 24.11.2021
(51) Int. Cl.: A61Q 1/02, A61Q 17/04, A61Q 19/00, A61K 8/19, A61K 8/81, A61K 8/85, A61K 8/87

(54) **FILM-FORMING COMPOSITION FOR SKIN**

(30) Priority: 30.11.2020 JP 2020199006
(71) Applicant: Kao Corporation, Chuo-ku Tokyo 103-8210 (JP)
(72) Inventor: SHIROTSUKI, Sanae, Odawara-shi, Kanagawa 250-0002 (JP); ISHIDA, Kaori, Haga-gun, Tochigi 321-3497 (JP)
(74) Representative: Hoffmann Eitle
(86) International application number: PCT/JP2021/043012
(87) International publication number: WO 2022/114007

(57) **Abstract**

Provided is a film-forming composition for skin that can form a stable film on skin, makes the finish of skin bright, and make the roughness of the skin texture and the pores inconspicuous. The present invention relates to a film-forming composition for skin including the following components (A) and (B): (A) a powder; and (B) a fiber having an average fiber diameter of 0.1 µm or more and 7 µm or less and an aspect ratio (average fiber length/average fiber diameter) of 20 or more and 300 or less in an amount of 0.1 mass% or more and 10 mass% or less relative to the whole film-forming composition, in which the mass ratio of the component (B) to the component (A), (B/A), is 0.02 or more and 0.4 or less.

## Description

### Field of the Invention

The present invention relates to a film-forming composition for skin that can form a favorable cosmetic film on a skin surface.

### Background of the Invention

Techniques for incorporating fibers into cosmetics are well known and are broadly adopted for mascara and so on. In order to make up a keratinous substance, such as skin, a composition containing a fiber and a copolymer including a carboxylate group and a polydimethylsiloxane group in a physiologically acceptable medium (Patent Literature 1) has been reported. A technique of incorporating a fiber in a cosmetic for reducing the irritation of a cosmetic including an irritative component (Patent Literature 2) and a cosmetic including a fiber and an anti-aging agent for camouflaging skin imperfections and treating signs of skin aging (Patent Literature 3) have been also reported. Furthermore, it has been reported to blend a fiber dispersion with a monofilament diameter of 1 to 500 nm and the sum Pa of the monofilament ratio of 60% or more, in order to obtain a blended solution, emulsion, or gel excellent in uniform dispersibility and long-term dispersion stability (Patent Literature 4). Furthermore, a cosmetic containing a short fiber obtained by cutting a hyperfine synthetic fiber having a diameter of about 2 µm into a length of 5 to 50 µm has been reported (Patent Literature 5).

Patent Literature 1: JP-A-2002-193746
Patent Literature 2: JP-A-2002-293718
Patent Literature 3: JP-A-2002-293731
Patent Literature 4: JP-A-2005-320506
Patent Literature 5: JP-A-2001-64153

### Summary of the Invention

The present invention relates to a film-forming composition for skin, comprising the following components (A) and (B):
(A) a powder; and
(B) a fiber having an average fiber diameter of 0.1 µm or more and 7 µm or less and an aspect ratio (average fiber length/average fiber diameter) of 20 or more and 300 or less in an amount of 0.1 mass% or more and 10 mass% or less relative to the whole film-forming composition, wherein
   the mass ratio of the component (B) to the component (A), (B/A), is 0.02 or more and 0.4 or less.

The present invention relates to a method for producing a film on a skin surface, comprising applying the film-forming composition for skin to the skin.

Furthermore, the present invention relates to a film comprising the film-forming composition for skin.

### Brief Description of Drawing

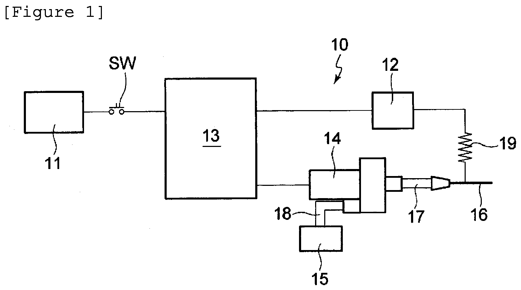

[Fig. 1] Figure 1 is a schematic diagram showing a structure of the electrostatic spraying device used for forming a fiber of a component (B).

### Detailed Description of the Invention

Since the fiber diameters adopted in Patent Literatures 1 to 3 are large, i.e., 0.9 dtex (= 10.7 µm) and the content of fiber is low, no network is formed by the fiber, and thus it causes a problem in the durability of cosmetic films. Since the fiber used in Patent Literature 4 has a long fiber length, the aspect ratio is significantly large, and a film having high durability may not be formed. Further, the aspect ratio of the fiber used in cosmetics disclosed in Patent Literature 5 is too small to form a stable film on skin.

According to studies by the present inventors, it was found that cosmetics in which a fiber having a large fiber diameter as disclosed in Patent Literatures 1 to 3 and a powder for cosmetics are blended have a problem of making the skin dark and dull, as well as making the roughness of the skin texture and the pore conspicuous.

Therefore, the present invention provides a film-forming composition for skin that can form a stable film on skin, make the finish of skin bright, and make the roughness of the skin texture and the pores inconspicuous.

The present inventors conducted various studies to solve the above problems. As a result, they found that a film-forming composition for skin prepared by blending a fine short fiber having a predetermined fiber diameter and aspect ratio and a powder at a predetermined ratio can form a stable film on skin. The fiber spreads over the sulci cutis and the cristae cutis and the powder adheres to the fiber, thereby making the finish of the skin bright and making the roughness of the skin texture and the pore inconspicuous.

According to the film-forming composition for skin of the present invention, a stable film can be formed on skin. The fiber spreads over the sulci cutis and the cristae cutis and the powder adheres to the fiber, thereby making the finish of skin bright and making the roughness of the skin texture and the pore inconspicuous. In addition, the roughness of the skin texture and the pore can be wholly covered by attaching both the film-forming composition for skin of the present invention and powdery foundation to the skin, imparting a smooth finish thereto.

The film-forming composition for skin of the present invention contains the following components (A) and (B):
(A) a powder; and
(B) a fiber having an average fiber diameter of 0.1 µm or more and 7 µm or less and an aspect ratio (average fiber length/average fiber diameter) of 20 or more and 300 or less in an amount of 0.1 mass% or more and 10 mass% or less relative to the whole film-forming composition, in which
   the mass ratio of the component (B) to the component (A), (B/A), is 0.02 or more and 0.4 or less.

The powder of the component (A) is a component providing various cosmetic effects to the film formed using the film-forming composition of the present invention on skin. Such a powder (A) is a solid other than the component (B) and can significantly make the finish of skin bright and make the roughness of the skin texture and the pores inconspicuous when used together with the component (B) compared when it is used as an ordinary powder-containing cosmetic or when it is used together with a conventional fiber having a large fiber diameter.

The powder of the component (A) is not particularly limited as long as it is a powder for cosmetics, and a color pigment or an extender pigment can be used. Among them, from the viewpoint of obtaining an excellent makeup effect, it is preferable to contain a color pigment. Here, examples of the color pigment (A1) include an inorganic color pigment, an inorganic white pigment, an organic color pigment, and an organic dye and also include a pearl pigment (brilliant powder).

Examples of the inorganic color pigment contained in the present invention composition specifically include inorganic color pigments, such as red iron oxide, iron hydroxide, iron titanate, yellow iron oxide, black iron oxide, carbon black, Prussian blue, ultramarine blue, Prussian blue titanium oxide, black titanium oxide, a titanium/titanium oxide sintered product, manganese violet, cobalt violet, chromium oxide, chromium hydroxide, cobalt oxide, and cobalt titanate. Examples of the inorganic white pigment include titanium oxide, zinc oxide, calamine, zirconium oxide, magnesium oxide, cerium oxide, aluminum oxide, and composites thereof.

One or two or more of these pigments can be used.

Among them, preferred are at least one or two or more selected from the group consisting of iron oxide, titanium oxide, and zinc oxide, and more preferred one or two or more selected from the group consisting of titanium oxide, zinc oxide, red iron oxide, yellow iron oxide, and black iron oxide.

Examples of the organic color pigment and organic dye include organic tar pigments, such as Red No. 3, Red No. 102, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1, and Blue No. 404; and organic dyes, such as β-carotene, caramel, and a paprika dye. In addition, the examples include those coated with polymers such as cellulose or polymethacrylate ester.

Examples of the pearl pigment (brilliant powder) include fish scale foil, titanium oxide-coated mica (mica titanium), bismuth oxychloride, titanium oxide-coated bismuth oxychloride, titanium oxide-coated talc, titanium oxide-coated colored mica, titanium oxide/iron oxide-coated mica, fine particle titanium oxide-coated mica titanium, fine particle zinc oxide-coated mica titanium, organic pigment-treated mica titanium, low-order titanium oxide-coated mica, titanium oxide-coated synthetic mica, titanium oxide-coated plate-like silica, hollow plate-like titanium oxide, iron oxide-coated mica, plate-like iron oxide (MIO), aluminum flake, stainless steel flake, titanium oxide-coated plate-like alumina, glass flake, titanium oxide-coated glass flake, pearl shell, gold foil, a gold-deposited resin film, and a metal-deposited resin film. One or two or more of them can be used.

Examples of the extender pigment include inorganic extender pigments and organic extender pigments.

Examples of the inorganic extender pigment include barium sulfate, calcium sulfate, magnesium sulfate, magnesium carbonate, calcium carbonate, talc, mica, kaolin, sericite, silicic acid, silicic anhydride, aluminum silicate, magnesium silicate, magnesium aluminum silicate, calcium silicate, barium silicate, strontium silicate, metal tungstate, hydroxyapatite, vermiculite, clay, bentonite, montmorillonite, hectorite, smectite, zeolite, ceramic powder, dicalcium phosphate, alumina, silica, aluminum hydroxide, boron nitride, synthetic mica, synthetic sericite, metallic soap, and barium sulfate-treated mica. One or two or more of them can be used.

Examples of the organic extender pigment include a silicone rubber powder, a silicone resin-coated silicone rubber powder, polymethylsilsesquioxane, a polyamide powder, a nylon powder, a polyester powder, a polypropylene powder, a polystyrene powder, a polyurethane powder, a vinyl resin powder, a urea resin powder, a phenolic resin powder, a fluorine resin powder, a silicone resin powder, an acrylic resin powder, a melamine resin powder, a polycarbonate resin, a divinylbenzene/styrene copolymer, a silk powder, a wool powder, a cellulose powder, a long-chain alkyl phosphate metal salt, N-mono (long-chain) alkylacyl basic amino acid, and composites thereof. One or two or more of them can be used.

Composite powders of the inorganic powder and the organic powder can also be used.

The particle diameter of the powder is preferably 0.01 µm or more and 500 µm or less, more preferably 0.02 µm or more and 100 µm or less, further preferably 0.03 µm or more and 10 µm or less, and even more preferably 0.03 µm or more and 2 µm or less.

Examples of the shape of the powder include spherical, plate-like, granular, and amorphous. In the plate-like shape here, the aspect ratio (average length/average thickness) is preferably less than 20, more preferably less than 15, and further preferably less than 10.

The powders can also be used after hydrophobic treatment, and one or two or more of these powders hydrophobized can also be used. The hydrophobic treatment is not limited as long as it is treatment that is applied to ordinary powders for cosmetics, and may be performed using a surface treatment agent such as a silicone compound, an alkylsilane, metal soap, an amino acid compound, lecithin, organic titanate, a fluorine compound, an acrylic resin, a methacrylic resin, or a urethane resin by dry treatment, wet treatment, or the like.

As the hydrophobic treatment, preferred are surface treatment, for example, treatment with a silicone compound such as dimethylpolysiloxane, methyl hydrogen polysiloxane, cyclic silicone, or one-terminal or both-terminal trialkoxy group-modified organopolysiloxane; treatment with an alkylsilane such as methyltrimethoxysilane, ethyltrimethoxysilane, hexyltrimethoxysilane, caprylyl trimethoxysilane, or caprylyl triethoxysilane; treatment with metal soap such as aluminum stearate, aluminum myristate, zinc stearate, or magnesium stearate; treatment with an amino acid compound such as proline, hydroxyproline, alanine, glycine, sarcosine, glutamic acid, aspartic acid, lysine, or a derivative thereof; lecithin treatment; treatment with an organic titanate such as isopropyl titanium triisostearate; treatment with a fluorine compound such as perfluoroalkyl alkoxysilane, fluorine-modified silicone, perfluoropolyether, or perfluoroalkyl phosphate; and acrylic resin treatment, methacrylic resin treatment, and urethane resin treatment. In particular, powders surface-treated with a silicone compound, an alkylsilane, or an amino acid compound are more preferable.

The content of the component (A) powder in the present invention film-forming composition for skin is preferably 0.5 mass% or more, more preferably 1 mass% or more, and further preferably 5 mass% or more from the viewpoint of improving the durability of the film and the viewpoint of making the finish of skin bright where the film is formed and being capable of making the roughness of the skin texture and the pores inconspicuous. In addition, from the same viewpoint, the content is preferably 94 mass% or less, more preferably 60 mass% or less, further preferably 50 mass% or less, and even more preferably 40 mass% or less.

The component (A) powder preferably contains a color pigment (A1) as described above, and the content of the color pigment in the film-forming composition for skin of the present invention is preferably 0.1 mass% or more and 60 mass% or less from the viewpoint of obtaining an excellent makeup effect. The content is more preferably 0.3 mass% or more, further preferably 1 mass% or more and is more preferably 50 mass% or less, further preferably 40 mass% or less, and preferably 30 mass% or less.

The content mass ratio of the color pigment (A1) to the component (A), (A1/A), is preferably 0.3 or more, more preferably 0.4 or more, and further preferably 0.5 or more. In addition, from the same viewpoint, the mass ratio is preferably 1 or less, more preferably 0.95 or less, and further preferably 0.9 or less.

The component (B) is a fiber having an average fiber diameter 0.1 µm or more and 7 µm or less and an aspect ratio (average fiber length/average fiber diameter) of 20 or more and 300 or less. The component (B) can forms a network in the formed film, provides durability to the film, makes the finish of skin bright where the film is formed, and makes the roughness of the skin texture and the pores inconspicuous. The component (B) is present as a solid in the film-forming composition.

Incidentally, whether a fiber forms a network in the film or not can be verified by using a scanning electron microscope (SEM). The network is a state that fibers dispersed in the film have intersections with each other to have gaps between the fibers and is a state that the component contained in the film-forming composition is retained in the gaps. The intersection of fibers is preferably a state that, for example, one fiber has two or more intersections with other two or more fibers, and the fibers are involved with each other.

The average fiber diameter is generally the diameter of a cross-section of a fiber. Here, when the cross-section of a fiber is a circle, the fiber diameter is the diameter of the circle, and when the cross-section is an ellipse, the fiber diameter is the longitudinal diameter of the ellipse. The average fiber diameter of the fiber used in the present invention is 0.1 µm or more and 7 µm or less from the viewpoint of improving the followability of the fiber to the skin in the formed film and improving the durability and the viewpoint of making the finish of skin bright where the film is formed and making the roughness of the skin texture and the pores inconspicuous.

The average fiber diameter is preferably 0.2 µm or more and more preferably 0.3 µm or more from the viewpoint of improving the durability and the viewpoint of making the finish of skin bright where the film is formed and making the roughness of the skin texture and the pores inconspicuous.

The average fiber diameter is preferably 5 µm or less, more preferably 4 µm or less, and further preferably 3 µm or less from the viewpoint of improving the durability and the viewpoint of making the finish of skin bright where the film is formed and making the roughness of the skin texture and the pores inconspicuous.

The average fiber diameter can be measured by observing fibers with an SEM at a magnification of 2000 times or 5000 times, arbitrarily selecting, from the two-dimensional image, 100 fibers excluding defects (e.g., clumps of fibers and crossing parts of fibers), drawing a line perpendicular to the longitudinal direction of each of the fibers, and directly reading the fiber diameters. The arithmetic average of these measured values is determined as the average fiber diameter. Since the fibers are dispersed in the film-forming composition, the film-forming composition is thinly applied to a substrate, and measurement by SEM observation is performed.

The length of the fiber is preferably 20 µm or more and 300 µm or less as the average fiber length from the viewpoint of a length of facilitating network formation and improving the durability of the formed film by the network and the viewpoint of making the finish of skin bright where the film is formed and making the roughness of the skin texture and the pores inconspicuous.

The average fiber length is more preferably 25 µm or more, further preferably 30 µm or more, and further preferably 40 µm or more from the viewpoint of facilitating network formation and the viewpoint of making the finish of skin bright where the film is formed and making the roughness of the skin texture and the pores inconspicuous.

The average fiber length is more preferably 250 µm or less and further preferably 200 µm or less from the viewpoint of suppressing tangles and twists between fibers at the time of application of the composition and the viewpoint of making the finish of skin bright where the film is formed and making the roughness of the skin texture and the pores inconspicuous.

The average fiber length can be measured by observing fibers with an SEM at a magnification of 250 times to 750 times according to the length of the fibers, arbitrarily selecting, from the two-dimensional image, 100 fibers excluding defects (e.g., clumps of fibers and crossing parts of fibers), drawing a line in the longitudinal direction of each of the fibers, and directly reading the fiber lengths. The arithmetic average of these measured values is determined as the average fiber length.

The aspect ratio of the fiber (average fiber length)/(average fiber diameter) is preferably 20 or more and 300 or less from the viewpoint of the durability of the film by forming a uniform network and the viewpoint of making the finish of skin bright where the film is formed and making the roughness of the skin texture and the pores inconspicuous.

The aspect ratio is more preferably 25 or more, further preferably 27 or more, and further preferably 30 or more from the viewpoint of the durability of the film and the viewpoint of making the finish of skin bright where the film is formed and making the roughness of the skin texture and the pores inconspicuous.

The aspect ratio is more preferably 250 or less and further preferably 200 or less from the viewpoint of the durability of the film and the viewpoint of making the finish of skin bright where the film is formed and making the roughness of the skin texture and the pores inconspicuous.

The CV (coefficient of variation) value of the fiber length of the component (B) is preferably 40% or more and 100% or less from the viewpoint that the fiber forms a network in the film.

The CV value is more preferably 42% or more and further preferably 45% or more from the viewpoint of facilitating network formation and the viewpoint of making the finish of skin bright where the film is formed and making the roughness of the skin texture and the pores inconspicuous.

The CV value is preferably 95% or less and further preferably 90% or less from the viewpoint of enhancing the storage stability of the composition.

The CV value is calculated from the measured values obtained by the above-described fiber length-measuring method by the expression: (standard deviation of measured fiber lengths)/(average fiber length) × 100 (%).

The fiber of the component (B) preferably includes a fiber having a fiber length of 40 µm or more and more preferably a fiber having a fiber length of 50 µm or more from the viewpoint of forming a strong network in the film and enhancing the durability of the obtained film and the viewpoint of making the finish of skin bright where the film is formed and making the roughness of the skin texture and the pores inconspicuous.

In the fiber of the component (B), the rate of the number of fibers having a fiber length of 40 µm or more to the total fibers is preferably 5% or more and 100% or less from the viewpoint of forming a strong network in the film and enhancing the durability of the obtained film and the viewpoint of making the finish of skin bright where the film is formed and making the roughness of the skin texture and the pores inconspicuous. The fibers having a length of 40 µm or more is more preferably contained at a rate of 8% or more and 100% or less and is further preferably contained at a rate of 15% or more and 100% or less from the viewpoint of further improving the durability and the viewpoint of making the finish of skin bright where the film is formed and making the roughness of the skin texture and the pores inconspicuous.

This rate of the number of fibers is measured by adjusting the magnification of the SEM within ×200 to ×750 according to the fiber length such that 20 to 30 fibers are present in one imaging screen of the SEM and, in this state, measuring the fiber lengths of all fibers in the image for eliminating arbitrariness. The measurement is performed for 200 or more fibers in total.

The fiber of the component (B), i.e., the fiber of a water-insoluble polymer, can be manufactured by obtaining a fiber from a fiber-forming polymer by various known spinning technologies and subjecting the fiber for shortening treatment. Here, the fiber-forming polymer is usually a thermoplastic or solvent-soluble chain polymer molecule.

The fiber-forming polymer is preferably a thermoplastic resin and more preferably a thermoplastic resin having a weight-average molecular weight of 1.0 × 10⁴ g/mol or more and 2.0 × 10⁵ g/mol or less.

As the fiber-forming polymer, a water-insoluble polymer is preferably used for maintaining the form of the fiber in a film-forming agent. The spinning method is preferably an electrospinning method for efficiently obtaining a fiber having a small fiber diameter. Specifically, solution spinning and melt spinning are mentioned.

The term "fiber of a water-insoluble polymer" refers to a fiber having a property that when 1 g of the fiber is weighed in an environment of 23°C and 1 atm and is then immersed in 10 g of deionized water for 24 hours, the dissolved amount of the immersed fiber does not exceed 0.5 g.

Examples of the water-insoluble polymer include a completely saponified polyvinyl alcohol that can be insolubilized after film formation; a partially saponified polyvinyl alcohol that can be crosslinked after film formation by using a crosslinking agent together; oxazoline-modified silicone, such as a poly(N-propanoylethyleneimine) graft-dimethylsiloxane/y-aminopropylmethylsiloxane copolymer; biodegradable resins, such as polyvinylacetal diethylaminoacetate, zein (major component of corn protein), polylactic acid (PLA), polybutylene succinate, polyglycolic acid, polycaprolactone, and polyhydroxy alkanoic acid; polyester resins, such as polyethylene terephthalate (PET) and polybutylene terephthalate; acrylic resins, such as a polyacrylonitrile resin and a polymethacrylic acid resin; and a polystyrene resin, a polyvinylbutyral resin, a polyvinylacetal resin, a polyurethane resin, a polyamide resin, a polyimide resin, a polyamideimide resin, a polypropylene resin, a polyethylene resin, and various polypeptides (e.g., collagen, gelatin, fibrin, and casein). These water-insoluble polymers can be used singly or in combinations of two or more thereof.

Among these water-insoluble polymers, it is preferable to use one or two or more selected from the group consisting of a completely saponified polyvinyl alcohol that can be insolubilized after film formation, a partially saponified polyvinyl alcohol that can be crosslinked after film formation by using a crosslinking agent together, a polymethacrylic acid resin and other acrylic resins, a polyvinylacetal resin, a polyurethane resin, polylactic acid, an oxazoline-modified silicone such as a poly(N-propanoylethyleneimine) graft-dimethylsiloxane/y-aminopropylmethylsiloxane copolymer, polyvinylacetal diethylaminoacetate, and zein.

Among them, from the viewpoint of the ease in formation of nanofibers, more preferred are one or two or more selected from the group consisting of a polyvinylbutyral resin, an acrylic resin, a polypropylene resin, a polyester such as polylactic acid, and a polyurethane resin.

The acrylic resin is preferably an (octyl acrylamide/hydroxypropyl acrylate/butylaminoethyl methacrylate) copolymer.

In addition, it is also preferable from the viewpoint of the reduction in environmental load to use a biodegradable resin such as polylactic acid, polybutylene succinate, polyglycolic acid, polycaprolactone, and polyhydroxy alkanoic acid. In the present specification, the term "biodegradability" means that the degree of biodegradation of a polyester measured in accordance with JIS K6953-1 is 30% or more.

Examples of the means for fiber shortening treatment include cutting, shearing, crushing, pulverizing, disintegrating, and defibrating. For example, it is possible to use an impact crusher such as a mechanical vortex crusher or a hammer crusher, a jet grinder such as a jet mill, a medium grinder such as a ball mill or a rod mill, a dry grinder such as a cutter mill grinder or a disc mill grinder, a media grinder using a liquid medium, a wet grinder using a medialess grinder, or a combination thereof.

In more preferred means for shortening fibers, a fiber assembly in which nanofibers are entangled, e.g., nonwoven fabric, is manufactured, the fiber assembly is then cut into an appropriate size, and then a mechanical vortex grinder, a cutter mill grinder, a disc mill grinder, a wet high-speed shear medialess grinder, or a wet high-pressure shear medialess grinder is used. Examples of the fiber assembly include, in addition to nonwoven fabric, those having a prescribed thickness, such as flocculate.

The content of the component (B) in the present invention composition is 0.1 mass% or more and 10 mass% or less relative to the whole film-forming composition from the viewpoint of the durability of the formed film and the ease in formation of a fiber network and the viewpoint of making the finish of skin bright where the film is formed and making the roughness of the skin texture and the pores inconspicuous.

The content is preferably 0.5 mass% or more and more preferably 0.7 mass% or more from the viewpoint of the durability of the film and the ease in formation of a fiber network and the viewpoint of making the finish of skin bright where the film is formed and making the roughness of the skin texture and the pores inconspicuous.

In addition, from the viewpoint of forming a stable composition, the content is preferably 9 mass% or less and further preferably 8 mass% or less.

The content of the component (B) relative to the whole film-forming composition can be determined as follows. Firstly, a fiber of a water-insoluble polymer recognized by the above-described definition of a water-insoluble polymer is obtained from the fibers contained in the composition. Secondly, the fiber is washed with a solvent in which the fiber is insoluble, and only the fiber of a water-insoluble polymer is obtained through filtration. When the resin contained in the component (B) is an ester resin such as PLA, the solvent is preferably ethanol. When the resin is acrylic, the solvent is preferably water. The obtained fiber of a water-insoluble polymer can be measured to determine the mass. The rate relative to the mass of the composition before the washing, i.e., the whole film-forming composition, can be determined by (component (B) mass after washing)/(mass of composition before washing) × 100 (%) .

The mass ratio of the component (B) to the component (A), (B/A), in the present invention composition is 0.02 or more and 0.4 or less from the viewpoint of the durability of the film and the ease in formation of a fiber network and the viewpoint of making the finish of skin bright where the film is formed and making the roughness of the skin texture and the pores inconspicuous.

The mass ratio (B/A) is preferably 0.03 or more, more preferably 0.05 or more, and further preferably 0.1 or more from the viewpoint of the durability of the film and the ease in formation of a fiber network and the viewpoint of making the finish of skin bright where the film is formed and making the roughness of the skin texture and the pores inconspicuous.

The mass ratio (B/A) is preferably 0.35 or less from the viewpoint of the durability of the film and the ease in formation of a fiber network and the viewpoint of making the finish of skin bright where the film is formed and making the roughness of the skin texture and the pores inconspicuous and is more preferably 0.33 or less and further preferably 0.31 or less from the viewpoint of making the finish of skin bright where the film is formed and making the roughness of the skin texture and the pores inconspicuous.

In the composition of the present invention, in order to form a network in the formed film and improve the durability of the film, it is preferable that the (average fiber diameter) ²/fiber content (µm²/ mass%) is within a range of 0.005 or more and 7 or less. The fiber content means the mass% of the fiber in the film-forming composition.

This value is preferably 0.02 or more, more preferably 0.03 or more, and further preferably 0.05 or more from the viewpoint of sufficiently forming a uniform network of the fiber.

Considering the practical blending amount, the value is preferably 6 or less, more preferably 5 or less, and further preferably 4 or less.

This value, i.e., (average fiber diameter)²/fiber content (µm²/mass%) , is an index of the cumulative length of the fiber included in the composition and means that the larger this value, the shorter the cumulative length.

The present invention composition preferably contains a liquid material (component (C)) selected from the group consisting of water and nonvolatile oils that are liquid at 20°C as a dispersion medium of the component (B) in order to facilitate the formation of a network of the fiber of the component (B) in the film formed on skin when the present invention composition is applied to the skin.

Thus, in the present invention film-forming composition, since the component (B) is present dispersed or dissolved in the component (C), it becomes easy to form a network of the fiber of the component (B).

The component (C) is a liquid material selected from the group consisting of water and nonvolatile oils that are liquid at 20°C. Examples of the component (C) include water and one or two or more oils selected from the group consisting of ester oils, ether oils, hydrocarbon oils, higher alcohols, fluorine oils, and nonvolatile silicone oils. In the present invention, they are preferably used singly or in combinations of two or more thereof. The volatile oil in the present invention is an oil having a vapor pressure at 20°C of 0.01 kPa or more and 106.66 kPa or less, and the nonvolatile oil is an oil that is liquid at 20°C other than the volatile oils.

As the ester oil, it is possible to use one or two or more selected from the group consisting of esters consisting of linear or branched fatty acid and linear or branched alcohol or polyhydric alcohol and triglycerol fatty acid esters (triglycerides).

Specifically, it is possible to used one or two or more selected from the group consisting of isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, isononyl isononanoate, isotridecyl isononanoate, cholesteryl 12-hydroxystearate, ethylene glycol di-2-ethylhexanoate, dipentaerythritol fatty acid ester, n-alkyl glycol monoisostearate, neopentyl glycol dicaprate, diisostearyl malate, glyceryl di-2-heptylundecanoate, trimethylolpropane tri-2-ethylhexanoate, trimethylolpropane triisostearate, pentaerythrityl tetra-2-ethylhexanoate, glyceryl tri-2-ethylhexanoate, trimethylolpropane triisostearate, cetyl 2-ethylhexanoate, 2-ethylhexyl palmitate, diethylhexyl naphthalenedicarboxylate, C12-15 alkyl benzoate, cetearyl isononanoate, glyceryl tri(caprylate/caprate), butylene glycol (dicaprylate/caprate), glyceryl trilaurate, glyceryl trimyristate, glyceryl tripalmitate, glyceryl triisostearate, glyceryl tri-2-heptylundecanoate, glyceryl tribehenate, tricoconut oil fatty acid glyceryl ester, castor oil fatty acid methyl ester, oleyl oleate, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, di-2-ethylhexyl succinate, triethyl citrate, ethylhexyl para-methoxycinnamate, and tripropylene glycol dipivalate.

Among them, from the viewpoint of the durability of the formed film, the viewpoint of the ease in formation of a fiber network, and the viewpoint of making the finish of skin bright where the film is formed and making the roughness of the skin texture and the pores inconspicuous, preferred are at least one selected from the group consisting of octyldodecyl myristate, myristyl myristate, isocetyl stearate, isocetyl isostearate, cetearyl isononanoate, diisobutyl adipate, di-2-ethylhexyl sebacate, isopropyl myristate, isopropyl palmitate, diisostearyl malate, neopentyl glycol dicaprate, isononyl isononanoate, isotridecyl isononanoate, glyceryl tri(caprylate/caprate), isopropyl myristate, and ethylhexyl para-methoxycinnamate, and it is more preferable to include one or two or more selected from the group consisting of diisostearyl malate, neopentyl glycol dicaprate, isononyl isononanoate, isotridecyl isononanoate, glyceryl tri(caprylate/caprate), isopropyl myristate, and ethylhexyl para-methoxycinnamate.

Examples of the ether oil include alkyl-1,3-dimethylbutyl ether such as cetyl dimethylbutyl ether, ethylene glycol dioctyl ether, glycerol monooleyl ether, and dicaprylyl ether, and one or two or more selected therefrom can be used.

Examples of the hydrocarbon oil include hydrocarbon oils that are liquid at 20°C, such as liquid paraffin, squalane, squalene, polyisobutene (pentamer or higher), and liquid isoparaffin.

Examples of the higher alcohol include higher alcohols having from 12 to 20 carbon atoms, specifically, lauryl alcohol, isostearyl alcohol, oleyl alcohol, and octyldodecanol, and one or two or more selected therefrom can be used.

Furthermore, animal and vegetable oils including the above-mentioned ester oils and hydrocarbon oils can be used. Examples of the animal and vegetable oil include olive oil, jojoba oil, macadamia nut oil, meadowfoam oil, castor oil, safflower oil, sunflower oil, avocado oil, canola oil, apricot kernel oil, rice germ oil, and rice bran oil.

Examples of the silicone oil include dimethylpolysiloxane (5 cs or more), polyether-modified silicone, amino-modified silicone, carboxy-modified silicone, methylphenylpolysiloxane, fatty acid-modified silicone, alcohol-modified silicone, aliphatic alcohol-modified silicone, epoxy-modified silicone, fluorine-modified silicone, cyclic silicone, and alkyl-modified silicone. As the silicone oil, it is preferable to use at least dimethylpolysiloxane (5 cs or more).

Examples of the fluorine oil include perfluorodecaline, perfluoroadamantane, perfluorobutyl tetrahydrofuran, perfluorooctane, perfluorononane, perfluoropentane, perfluorodecane, perfluorododecane, and perfluoropolyether.

The content of the component (C) in the present invention composition is preferably 5 mass% or more, more preferably 10 mass% or more, further preferably 15 mass% or more, and even more preferably 20 mass% or more considering the practical blending amount from the viewpoint of the dispersibility of the component (B) and the durability of the formed film.

In addition, considering the practical blending amount, the content is preferably 98 mass% or less, more preferably 90 mass% or less, further preferably 70 mass% or less, and even more preferably 50 mass% or less.

The content and skeletal structure of the component (C) can be identified by specifying the molecular structure by a known technology, such as NMR (nuclear magnetic resonance apparatus), chromatography, or IR analysis, or a combination thereof. The content of the component (C) can be measured by the measurement method above, for example, based on the intensity of the measured value of the portion showing the skeletal structure.

The content of water (C1) of the component (C) in the film-forming composition is preferably 1 mass% or more, more preferably 5 mass% or more, further preferably 10 mass% or more, and even more preferably 15 mass% or more and is preferably 98 mass% or less, more preferably 90 mass% or less, further preferably 70 mass% or less, and even more preferably 50 mass% or less from the viewpoint of the durability of the film, the viewpoint of the ease in formation of a fiber network, and the viewpoint of making the finish of skin bright where the film is formed and making the roughness of the skin texture and the pores inconspicuous.

The mass ratio of the component (C1) to the component (C), (C1)/(C), is preferably 0.4 or more, more preferably 0.5 or more, further preferably 0.6 or more, and even more preferably 0.7 or more and is preferably 0.96 or less, more preferably 0.9 or less, further preferably 0.85 or lee, and even more preferably 0.8 or less from the viewpoint of film formation and the durability of the film, the viewpoint of the ease in formation of a fiber network, and the viewpoint of forming a film excellent in scratch resistance.

The film-forming composition for skin of the present invention can further contain an oil other than the component (C), a volatile component, a surfactant, a polyol that is liquid at 20°C, a preservative, a moisturizing agent, an ultraviolet absorber, a watersoluble polymer, an amino acid, a dye, and so on.

The oil other than the component (C) is not limited as long as it is solid or semisolid at 20°C and is used in ordinary cosmetics, and examples thereof include mineral-based waxes such as ozokerite and ceresin; petroleum waxes such as paraffin and microcrystalline wax; synthetic hydrocarbons such as Fischer-Tropsch wax, polyethylene wax, and synthetic hydrocarbon wax; vegetable waxes such as carnauba wax, candelilla wax, rice wax, sunflower wax, and highly hydrogenated jojoba oil; animal waxes such as beeswax Chinese wax, and whale wax; and synthetic waxes such as silicone wax and synthetic beeswax.

Examples of the volatile component (D) include alcohols, ketones, volatile silicone oils, and volatile hydrocarbon oils, and preferred are one or more selected from the group consisting of alcohols, volatile silicone, and volatile hydrocarbon oils. The volatile component is a material having volatility in the liquid state. The volatile material has a vapor pressure at 20°C of 0.01 kPa or more and 106.66 kPa or less.

As the volatile alcohol, for example, a monovalent chain aliphatic alcohol, a monovalent cyclic aliphatic alcohol, or a monovalent aromatic alcohol is suitably used. Examples of the monovalent chain aliphatic alcohol include C₁-C₆ chain alcohols, examples of the monovalent cyclic aliphatic alcohol include C₄-C₆ cyclic alcohols, and examples of the monovalent aromatic alcohol include benzyl alcohol and phenylethyl alcohol. Specific examples thereof include ethanol, isopropyl alcohol, butyl alcohol, phenylethyl alcohol, n-propanol, and n-pentanol. From the viewpoint of use impression, ethanol is preferable. These alcohols can be used alone or in combination of two or more.

Examples of the volatile silicone oil include linear dimethylpolysiloxanes, such as hexamethyldisiloxane (dimethylpolysiloxane (0.65 cs)), octamethyltrisiloxane (dimethylpolysiloxane (1 cs)), dimethylpolysiloxane (1.5 cs), and dimethylpolysiloxane (2 cs); branched siloxanes, such as methyl trimethicone, tris(trimethylsilyl)methylsilane, and tetrakis(trimethylsilyl)silane; and cyclic dimethylsiloxanes, such as octamethylcyclotetrasiloxane, decamethylcyclopentasiloxane, and dodecamethylcyclohexasiloxane.

Among them, from the viewpoint of excellent use impression and finish, preferred are linear dimethylpolysiloxanes and branched siloxane, and it is more preferable to include one or two or more selected from the group consisting of hexamethyldisiloxane (dimethylpolysiloxane (0.65 cs)), octamethyltrisiloxane (dimethylpolysiloxane (1 cs)), dimethylpolysiloxane (1.5 cs), dimethylpolysiloxane (2 cs), and methyl trimethicone, further preferable to at least include one or two or more selected from the group consisting of hexamethyldisiloxane (dimethylpolysiloxane (0.65 cs)), octamethyltrisiloxane (dimethylpolysiloxane (1 cs)), and methyl trimethicone, and even more preferable to at least include one or two or more selected from the group consisting of hexamethyldisiloxane (dimethylpolysiloxane (0.65 cs)), and octamethyltrisiloxane (dimethylpolysiloxane (1 cs)).

Examples of the volatile hydrocarbon oil include paraffin-based hydrocarbon oils, such as n-decane, n-undecane, and n-dodecane; isoparaffin-based hydrocarbon oils, such as isodecane, isododecane, and hydrogenated polyisobutene; and cyclic paraffin hydrocarbon oils, such as cyclodecane and cyclododecane. Among them, from the viewpoint of excellent use impression and suppressing uneven attachment of the finish, preferred are isoparaffin-based hydrocarbon oils, more preferred are isoparaffin-based hydrocarbon oils having from 8 to 16 carbon atoms, further preferred are isoparaffin-based hydrocarbon oils having from 10 to 16 carbon atoms, and it is even more preferable to at least include isododecane.

As the volatile oil, it is preferable to include one or two or more selected from the group consisting of isododecane and dimethylpolysiloxane having a kinematic viscosity at 25°C of 2 cSt or less from the viewpoint of excellent use impression and finish. Incidentally, the kinematic viscosity can be measured using, for example, an Ubbelohde viscometer.

The content of the volatile component in the present invention composition is preferably 1 mass% or more relative to the whole film-forming composition, more preferably 10 mass% or more, further preferably 25 mass% or more and preferably 60 mass% or less, more preferably 50 mass% or less, and further preferably 45 mass% or less from the viewpoint of the durability of the formed film and the ease in formation of a fiber network and the viewpoint of making the finish of skin bright where the film is formed and making the roughness of the skin texture and the pores inconspicuous.

Examples of the surfactant include nonionic surfactants, anionic surfactants, and cationic surfactants, and particularly preferred are nonionic surfactants, such as a polyoxyethylene/methylpolysiloxane copolymer, a poly(oxyethylene/oxypropylene)methylpolysiloxane copolymer, crosslinked polyether-modified silicone, crosslinked alkylpolyether-modified silicone, cetyl dimethicone copolyol, sorbitan monooleate, glyceryl stearate, polyoxyethylene hydrogenated castor oil, polyoxyethylene alkyl ether, sorbitan sesquioleate, and diglyceryl monooleate. These surfactants may be used singly or in combinations of two or more thereof.

The content of the surfactant in the present invention composition is preferably 0.01 mass% or more relative to the whole film-forming composition, more preferably 0.1 mass% or more, and further preferably 0.3 mass% or more and preferably 10 mass% or less, more preferably 5 mass% or less, and further preferably 3 mass% or less from the viewpoint of the durability of the formed film and the ease in formation of a fiber network and the viewpoint of making the finish of skin bright where the film is formed and making the roughness of the skin texture and the pores inconspicuous.

Examples of the polyol that is liquid at 20°C include alkylene glycols, such as ethylene glycol, propylene glycol, 1,3-propanediol, and 1,3-butanediol; polyalkylene glycols, such as diethylene glycol, dipropylene glycol, and polyethylene glycol and polypropylene glycol each having a weight-average molecular weight of 2000 g/mol or less; and glycerins, such as glycerin, diglycerin, and triglycerin.

Among them, preferred are ethylene glycol, propylene glycol, 1,3-butanediol, dipropylene glycol, polyethylene glycol having a weight-average molecular weight of 2000 g/mol or less, glycerin, and diglycerin; more preferred are propylene glycol, 1,3-butanediol, and glycerin; and further preferred are propylene glycol and 1,3-butanediol.

Examples of the form of the composition of the present invention include oily cosmetics and emulsified cosmetics, specifically, oily cosmetics, water-in-oil emulsified cosmetics, and oil-in-water emulsified cosmetics. In particular, emulsified cosmetics are preferable, and water-in-oil emulsified cosmetics are more preferable. The composition of the present invention can be manufactured by heating the components as needed and mixing them according to a usual method.

The film-forming composition for skin of the present invention can be applied as, for example, makeup cosmetics, such as a makeup base, a foundation, a concealer, a blusher, an eyeshadow, a mascara, an eyeliner, an eyebrow, an overcoat agent, and a lipstick; ultraviolet protection cosmetics, such as a sunscreen emulsion and a sunscreen cream; and skincare cosmetics, such as a lotion, an emulsion, a cream, a serum, and a facial mask and is suitable as a makeup cosmetic or an ultraviolet protection cosmetic.

The composition of the present invention is a film-forming composition for skin and can form a uniform film on a skin surface by being applied to the skin. In this film, the fiber forms a network, as a result, the obtained film has excellent durability. The fiber spreads over the sulci cutis and the cristae cutis, and the powder adheres to the fiber. Consequently, the finish of the skin is brightened, and the roughness of the skin texture and the pores can be wholly covered. In addition, when the film-forming composition for skin of the present invention is attached to the skin together with a powdery foundation, the roughness of the skin texture and the pores can be wholly covered, and a smooth finish is obtained.

A powder-containing cosmetic film excellent in durability can be formed on a skin surface by applying the composition of the present invention to the skin. Examples of means for applying the composition to the skin include finger application, spray application, application using a tool such as a roller or sponge, and application in a form of a stick-shaped solid cosmetic.

The film formed on a skin surface by the present invention not only has excellent durability but also can make the finish of the skin bright and wholly cover the roughness of the skin texture and the pores by that the fiber spreads over the sulci cutis and the cristae cutis and the powder adheres to the fiber.

Here, the thickness of the film varies depending on the application amount and is, in a usual use (an application basis weight of 1 mg/cm² or more and 3 mg/cm² or less), preferably 0.3 µm or more and 30 µm or less and more preferably 0.5 µm or more and 20 µm or less. The thickness is measured after application to a substrate with a contact coating thickness gauge (manufactured by Mitutoyo Corporation, Litematic VL-50A) on the substrate. Incidentally, the substrate used here is made of PET.

Regarding the above-described embodiments, the present invention further discloses the following composition, producing method, and film.

<1> A film-forming composition for skin comprising the following components (A) and (B):
   (A) a powder; and
   (B) a fiber having an average fiber diameter of 0.1 µm or more and 7 µm or less and an aspect ratio (average fiber length/average fiber diameter) of 20 or more and 300 or less in an amount of 0.1 mass% or more and 10 mass% or less relative to the whole film-forming composition, wherein
      the mass ratio of the component (B) to the component (A), (B/A), is 0.02 or more and 0.4 or less.
<2> The film-forming composition for skin according to <1>, wherein the component (B) is a fiber of a water-insoluble polymer.
<3> The film-forming composition for skin according to <1> or <2>, wherein the component (B) has an aspect ratio (average fiber length/average fiber diameter) of 25 or more and 300 or less.
<4> The film-forming composition for skin according to any of <1> to <3>, wherein the content of the component (A) relative to the whole film-forming composition is 0.5 mass% or more and 98 mass% or less.
<5> The film-forming composition for skin according to any of <1> to <4>, further containing a component (C) nonvolatile liquid material.
<6> The film-forming composition for skin according to any of <1> to <5>, wherein the component (A) is one or more selected from the group consisting of extender pigments and color pigments and preferably includes a color pigment.
<7> The film-forming composition for skin according to any of <1> to <6>, wherein the component (A) includes one or more color pigments selected from the group consisting of inorganic color pigments, inorganic white pigments, organic color pigments, organic dyes, and pearl pigments (brilliant powders).
<8> The film-forming composition for skin according to any of <1> to <7>, wherein the component (A) contains a color pigment, and the content of the color pigment in the film-forming composition is 0.1 mass% or more and 60 mass% or less.
<9> The film-forming composition for skin according to any of <1> to <8>, wherein the content mass ratio of the color pigment (A1) to the component (A), (A1/A), is preferably 0.3 or more, more preferably 0.4 or more, and further preferably 0.5 or more and is preferably 1 or less, more preferably 0.95 or less, and further preferably 0.9 or less.
<10> The film-forming composition for skin according to any of <1> to <9>, wherein the component (B) has an average fiber diameter of 0.2 µm or more and 5 µm or less, preferably 0.3 µm or more and 4 µm or less, and further preferably 0.3 µm or more and 3 µm or less.
<11> The film-forming composition for skin according to any of <1> to <10>, wherein the component (B) is a fiber of a water-insoluble polymer.
<12> The film-forming composition for skin according to any of <1> to <11>, wherein the component (B) is a fiber including one or two or more polymers selected from the group consisting of a completely saponified polyvinyl alcohol that can be insolubilized after film formation, a partially saponified polyvinyl alcohol that can be crosslinked after film formation by using a crosslinking agent together, acrylic resins such as polymethacrylic acid resin, a polyvinylbutyral resin, a polyurethane resin, polylactic acid, an oxazoline-modified silicone such as a poly(N-propanoylethyleneimine) graft-dimethylsiloxane/y-aminopropylmethylsiloxane copolymer, polyvinylacetal diethylaminoacetate, and Zein; and is preferably a fiber including one or two or more polymers selected from the group consisting of a polyvinylbutyral resin, an acrylic resin, a polypropylene resin, a polyurethane resin, polylactic acid, polybutylene succinate, polyglycolic acid, polycaprolactone, and polyhydroxy alkanoic acid.
<13> The film-forming composition for skin according to any of <1> to <12>, wherein the component (B) is a fiber including an (octyl acrylamide/hydroxypropyl acrylate/butylaminoethyl methacrylate) copolymer.
<14> The film-forming composition for skin according to any of <1> to <13>, wherein the component (B) has an average fiber length of 20 µm or more and 300 µm or less, preferably 25 µm or more and 250 µm or less, more preferably 30 µm or more and 200 µm or less, and further preferably 40 µm or more and 200 µm or less.
<15> The film-forming composition for skin according to any of <1> to <14>, wherein the component (B) has an aspect ratio (average fiber length/average fiber diameter) of 25 or more and 300 or less, preferably 27 or more and 250 or less, and more preferably 30 or more and 200 or less.
<16> The film-forming composition for skin according to any of <1> to <15>, wherein the CV value of the fiber length of the component (B) is 40% or more and 100% or less, preferably 42% or more and 95% or less, and more preferably 45% or more and 90% or less.
<17> The film-forming composition for skin according to any of <1> to <16>, wherein the component (B) includes a fiber having an average fiber length of 40µm or more, preferably 50 µm or more.
<18> The film-forming composition for skin according to any of <1> to <17>, wherein the (average fiber diameter)²/fiber content (µm²/ mass%) in the composition is 0.02 or more and 7 or less, preferably 0.02 or more and 6 or less, more preferably 0.03 or more and 5 or less, and further preferably 0.05 or more and 4 or less.
<19> The film-forming composition for skin according to any of <1> to <18>, wherein the content of the component (B) is 0.1 mass% or more and 10 mass% or less, preferably 0.5 mass% or more and 9 mass% or less, more preferably 0.7 mass% or more and 8 mass% or less.
<20> The film-forming composition for skin according to any of <1> to <19>, wherein the mass ratio of the component (B) to the component (A), (B/A), is 0.02 or more and 0.4 or less, preferably 0.03 or more and 0.35 or less, more preferably 0.05 or more and 0.33 or less, and further preferably 0.1 or more and 0.31 or less.
<21> The film-forming composition for skin according to any of <5> to <20>, wherein the component (C) is preferably one or more selected from the group consisting of water and oils that are liquid at 20°C.
<22> The film-forming composition for skin according to any of <5> to <20>, wherein the component (C) is water and one or two or more oils selected from the group consisting of ester oils, ether oils, hydrocarbon oils, higher alcohols, fluorine oils, and nonvolatile silicone oils.
<23> The film-forming composition for skin according to any of <5> to <21>, wherein the component (B) is preferably dispersed in the component (C).
<24> The film-forming composition for skin according to any of <1> to <23>, wherein the component (B) is a biodegradable resin.
<25> A method for producing a film on a skin surface, comprising applying the film-forming composition according to any of <1> to <24> to the skin.
<26> A film including the film-forming composition for skin according to any of <1> to <24>.
<27> Use of the film-forming composition according to any one of <1> to <24> as an emulsified cosmetic.
<28> Use of the film-forming composition according to any one of <1> to <24> for makeup and/or ultraviolet protection by applying the composition to skin, preferably, to the face.
<29> Use of the film-forming composition for skin according to any of <1> to <24> for producing a powder-containing cosmetic film on a skin surface.

### Examples

The present invention will now be described in further detail with reference to examples.

### [Production example of component (B)]

A production example of a fiber B will be shown.
(1) An acrylic resin ((octyl acrylamide/hydroxypropyl acrylate/butylaminoethyl methacrylate) copolymer) was dissolved in ethanol to obtain an 18 mass% solution. A nanofiber sheet was formed on the surface of a collector using this solution with the device for an electrospinning method shown in Figure 1. The conditions for manufacturing the nanofiber are as follows:
   Applied voltage: 30 kV; Capillary-collector distance: 150 mm; Aqueous solution discharge amount: 12 mL/hour; and Environment: 25°C, 30%RH.
(2) The obtained nanofiber sheet was appropriately cut and was then pulverized with an agitation system (manufactured by PRIMIX Corporation, LABOLUTION (registered trademark)) attached with dispersal blades at a rotational speed of 5000 rpm for 30 minutes to obtain a fiber B.

Fibers A and C to F were manufactured as with the fiber B by changing the polymer concentration, rotational speed, and shearing time.

A production example of a fiber G will be shown.
(1) An ester resin (polylactic acid) was dissolved in a mixture of chloroform and dimethylformamide (mass ratio = 80 : 20) to obtain a 20 mass% solution. A nanofiber sheet was formed on the surface of a collector, by using this solution with the device for an electrospinning method shown in Figure 1. The conditions for manufacturing the nanofiber are as follows:
   Applied voltage: 30 kV; Capillary-collector distance: 150 mm; Aqueous solution discharge amount: 12 mL/hour; and Environment: 25°C, 30%RH.
(2) The obtained nanofiber sheet was sheared using a dispersion device (manufactured by Pacific Machinery & Engineering Co., Ltd, Milder) at 13500 rpm and circulating 8 times in a circulation line to obtain a fiber.

Fiber H was manufactured as in the fiber G by changing the polymer concentration and circulation number.

As the fiber I, White nylon fiber 0.5-6D manufactured by Cosmeterials, Inc. was used.

### [Production example of composition]

Water-in-oil emulsified compositions were obtained by blending the obtained fibers and the components shown in Tables 1 to 4.

### [Examples 1 to 12 and Comparative Examples 1 to 4]

The finish of the base was evaluated by applying the water-in-oil emulsified compositions shown in Tables 1 to 4 to a pore model at 1 mg/cm². In addition, the finish when a powdery foundation was applied in the same amount was also evaluated. The evaluation was performed by comparing with the cosmetic of the reference prescription shown in Table 5 as follows. The results are shown in Tables 1 to 4.

### (Evaluation model)

The cosmetic of the reference prescription (Table 5) and the cosmetics shown in Tables 1 to 4 were applied to a pore model (manufactured by Beaulax Co., Ltd., cheek skin model No. 10A) in half. After the application, the brightness was visually evaluated (N = 5, comparison with the reference prescription).

Subsequently, a powdery foundation (Sofina Alblanc: Transclear White Foundation UV powder) was applied, and the roughness covering effect and the smooth finish were visually evaluated (N = 5).

The evaluation criteria were that when a cosmetic of Tables 1 to 4 was superior to the reference prescription, the score is 1 point; and when there was no difference or when a cosmetic of Tables 1 to 4 was inferior to the reference prescription, the score is 0 point. A highest score is 5 points.

**[Table 1]**

| | | | | | Example 1 | Example 2 | Example 3 | Example 4 | Example 5 | Example 6 | Example 7 |
|---|---|---|---|---|---|---|---|---|---|---|---|
| | | | | | Fiber A | Fiber B | Fiber C | Fiber B | Fiber D | Fiber B | Fiber E |
| | | (B) Fiber | | Polymer material | Acrylic resin | Acrylic resin | Acrylic resin | Acrylic resin | Acrylic resin | Acrylic resin | Acrylic resin |
| | | | | Fiber diameter (µm) | 0.3 | 0.6 | 0.6 | 0.6 | 0.6 | 0.6 | 1.8 |
| | | | | Fiber length (µm) | 50 | 50 | 20 | 50 | 100 | 50 | 50 |
| | | | | Aspect ratio | 166.7 | 83.3 | 33.3 | 83.3 | 166.7 | 83.3 | 27.8 |
| | | | | Rate of the number of fibers having a fiber length of 40 µm or more | 52.3 | 54.3 | 10.1 | 54.3 | 78.3 | 54.3 | 70.9 |
| | | | | | Example 1 | Example 2 | Example 3 | Example 4 | Example 5 | Example 6 | Example 7 |
| | | (B) Type of fiber | | | Acrylic resin | Acrylic resin | Acrylic resin | Acrylic resin | Acrylic resin | Acrylic resin | Acrylic resin |
| Powder phase | | (A) Powder | Color pigment | Silicone-treated yellow iron oxide | 0.01 | 0.01 | 0.01 | 0.01 | 0.01 | 0.01 | 0.01 |
| | | | | Silicone-treated red iron oxide | 0.03 | 0.03 | 0.03 | 0.03 | 0.03 | 0.03 | 0.03 |
| | | | | Silicone-treated black iron oxide | 0.001 | 0.001 | 0.001 | 0.001 | 0.001 | 0.001 | 0.001 |
| | | | | Silicone-treated fine particle zinc oxide (manufactured by TAYCA Corp. MZY-505M) 25 nm | 9 | 9 | 9 | 9 | 9 | 9 | 9 |
| | | | | Silicone-treated fine particle titanium oxide (manufactured by TAYCA Corp. MT-500SA) | 1 | 1 | 1 | 1 | 1 | 1 | 1 |
| | | | | Triethoxycaprylylsilane-treated fine particle titanium oxide (manufactured by Sakai Chemical Industry Co., Ltd., STR-100W) | 4 | 4 | 4 | 4 | 4 | 4 | 4 |
| | | | | Silicone-treated titanium oxide (manufacture by ISHIHARA SANGYOU KAISHA, LTD., CR-50, average particle diameter: 0.25 µm) | 0.56 | 0.56 | 0.56 | 0.56 | 0.56 | 0.56 | 0.56 |
| | | | Extender pigment | Silicone-coated talc (JA-68R) | 2 | 2 | 2 | 2 | 2 | 2 | 2 |
| Oil phase | | (B) Fiber | | Each fiber above | 3 | 1 | 3 | 3 | 3 | 5 | 3 |
| | | (C) Water and nonvolatile oil that is liquid at 20°C | | Dimethylpolysiloxane KF-96A-1000CS (-G) | 5 | 5 | 5 | 5 | 5 | 5 | 5 |
| | | | | Octyl p-methoxycinnamate | 4 | 4 | 4 | 4 | 4 | 4 | 4 |
| Water phase | | | | Purified water | 25 | 28 | 25 | 25 | 25 | 20 | 25 |
| Oil phase | | Surfactant | | Polyether-modified silicone (DOWSIL SH3775M FLUID) | 2.5 | 2.5 | 2.5 | 2.5 | 2.5 | 2.5 | 2.5 |
| | | (D) Volatile oil | | Dimethylpolysiloxane (KF-96L-1.5CS) | 10.699 | 9.699 | 10.699 | 10.699 | 10.699 | 13.699 | 10.699 |
| | | | | Dimethylpolysiloxane (KF-96L-2CS) | 26.5 | 26.5 | 26.5 | 26.5 | 26.5 | 26.5 | 26.5 |
| Water phase | | Other component | | Ethanol | 6.5 | 6.5 | 6.5 | 6.5 | 6.5 | 6.5 | 6.5 |
| | | | | Magnesium sulfate | 0.2 | 0.2 | 0.2 | 0.2 | 0.2 | 0.2 | 0.2 |
| Total amount | | | | | 100 | 100 | 100 | 100 | 100 | 100 | 100 |
| (A) Powder total | | | | | 16.601 | 16.601 | 16.601 | 16.601 | 16.601 | 16.601 | 16.601 |
| (B) Fiber | | | | | 3 | 1 | 3 | 3 | 3 | 5 | 3 |
| (C) Water and nonvolatile oil that is liquid at 20°C | | | | | 34 | 37 | 34 | 34 | 34 | 29 | 34 |
| | Surfactant | | | | 2.5 | 2.5 | 2.5 | 2.5 | 2.5 | 2.5 | 2.5 |
| (D) Volatile oil | | | | | 37.199 | 36.199 | 37.199 | 37.199 | 37.199 | 40.199 | 37.199 |
| (B) (Average fiber diameter)²/fiber content | | | | | 0.03 | 0.36 | 0.12 | 0.12 | 0.12 | 0.07 | 1.08 |
| (A1) Color pigment | | | | | 14.601 | 14.601 | 14.601 | 14.601 | 14.601 | 14.601 | 14.601 |
| (A1)/(A) | | | | | 0.88 | 0.88 | 0.88 | 0.88 | 0.88 | 0.88 | 0.88 |
| (B)/(A) | | | | | 0.181 | 0.060 | 0.181 | 0.181 | 0.181 | 0.301 | 0.181 |
| Evaluation result | | | Base application amount 1 mg/cm² | (1) Finish of base: bright and toned (Evaluation N = 5 out of 5) | 5 | 4 | 4 | 4 | 3 | 5 | 4 |
| | | | With powdery foundation in the same amount | (2) Base + powdery foundation finish: covering roughness and pores (Evaluation N = 5 out of 5) | 5 | 4 | 5 | 4 | 4 | 5 | 4 |
| | | | | (3) Base + powdery foundation finish: smooth finish (Evaluation N = 5 out of 5) | 5 | 4 | 5 | 4 | 4 | 5 | 4 |

**[Table 2]**

| | | | | | Example 8 | Example 9 | Example 10 | Comparative Example 1 | Comparative Example 2 | Comparative Example 3 | Comparative Example 4 |
|---|---|---|---|---|---|---|---|---|---|---|---|
| | | | | | Fiber F | Fiber G | Fiber G | Fiber H | Fiber B | Fiber B | Fiber I |
| | | (B) Fiber | | Polymer material | Acrylic resin | PLA | PLA | PLA | Acrylic resin | Acrylic resin | Nylon resin |
| | | | | Fiber diameter (µm) | 5 | 1.5 | 1.5 | 10 | 0.6 | 0.6 | 20 |
| | | | | Fiber length (µm) | 50 | 50 | 50 | 50 | 50 | 50 | 500 |
| | | | | Aspect ratio | 10.0 | 33.3 | 33.3 | 5.0 | 83.3 | 83.3 | 25.0 |
| | | | | Rate of the number of fibers having a fiber length of 40 µm or more | 80.4 | 40.0 | 40.0 | 77.3 | 54.3 | 54.3 | |
| | | | | | Example 8 | Example 9 | Example 10 | Comparative Example 1 | Comparative Example 2 | Comparative Example 3 | Comparative Example 4 |
| | | (B) Type of fiber | | | Acrylic resin | PLA | PLA | PLA | Acrylic resin | Acrylic resin | Nylon resin |
| Powder phase | | (A) Powder | Color pigment | Silicone-treated yellow iron oxide | 0.01 | 0.01 | 0.01 | 0.01 | 0.01 | 0.01 | 0.01 |
| | | | | Silicone-treated red Iron oxide | 0.03 | 0.03 | 0.03 | 0.03 | 0.03 | 0.03 | 0.03 |
| | | | | Silicone-treated black Iron oxide | 0.001 | 0.001 | 0.001 | 0.001 | 0.001 | 0.001 | 0.001 |
| | | | | Silicone-treated fine particle zinc oxide (manufactured by TAYCA Corp. MZY-505M) 25 nm | 9 | 9 | 9 | 9 | 9 | 9 | 9 |
| | | | | Silicone-treated fine particle titanium oxide (manufactured by TAYCA Corp. MT-500SA) | 1 | 1 | 1 | 1 | 1 | 1 | 1 |
| | | | | Triethoxycaprylylsilane-treated fine particle titanium oxide (manufactured by Sakai Chemical Industry Co., Ltd., STR-100W) | 4 | 4 | 4 | 4 | 4 | 4 | 4 |
| | | | | Silicone-treated titanium oxide (manufacture by ISHIHARA SANGYOU KAISHA, LTD., CR-50, average particle diameter: 0.25 µm) | 0.56 | 0.56 | 0.56 | 0.56 | 0.56 | 0.56 | 0.56 |
| | | | Extender pigment | Silicone-coated talc (JA-68R) | 2 | 2 | 2 | 2 | 2 | 2 | 2 |
| Oil phase | | (B) Fiber | | Each fiber above | 3 | 3 | 1 | 3 | 0.02 | 7 | 3 |
| | | (C) Water and nonvolatile oil that is liquid at 20°C | | Dimethylpolysiloxane KF-96A-1000CS (-G) | 5 | 5 | 5 | 5 | 5 | 5 | 5 |
| | | | | Octyl p-methoxycinnamate | 4 | 4 | 4 | 4 | 4 | 4 | 4 |
| Water phase | | | | Purified water | 23 | 23 | 25 | 25 | 30 | 15 | 23 |
| Oil phase | | Surfactant | | Polyether-modified silicone (DOWSIL SH3775M FLUID) | 2.5 | 2.5 | 2.5 | 2.5 | 2.5 | 2.5 | 2.0 |
| | | (D) Volatile oil | | Dimethylpolysiloxane (KF-96L-1.5CS) | 12.699 | 12.899 | 12.699 | 10.699 | 8.679 | 16.699 | 12.699 |
| | | | | Dimethylpolysiloxane (KF-96L-2CS) | 26.5 | 26.5 | 26.5 | 26.5 | 26.5 | 26.5 | 26.5 |
| Water phase | | Other component | | Ethanol | 6.5 | 6.5 | 6.5 | 6.5 | 6.5 | 6.5 | 6.5 |
| | | | | Magnesium sulfate | 0.2 | 0.2 | 0.2 | 0.2 | 0.2 | 0.2 | 0.2 |
| Total amount | | | | | 100 | 100 | 100 | 100 | 100 | 100 | 100 |
| (A) Powder total | | | | | 16.601 | 16.601 | 16.601 | 16.601 | 16.601 | 16.601 | 16.601 |
| (B) Fiber | | | | | 3 | 3 | 1 | 3 | 0.02 | 7 | 3 |
| (C) Water and nonvolatile oil that Is liquid at 20°C | | | | | 32 | 32 | 34 | 34 | 39 | 24 | 32 |
| | Surfactant | | | | 2.5 | 2.5 | 2.5 | 2.5 | 2.5 | 2.5 | 2.5 |
| (D) Volatile oil | | | | | 39.199 | 39.199 | 39.199 | 37.199 | 35.179 | 43.199 | 39.199 |
| (B) (Average fiber diameter)²/fiber content | | | | | 8.33 | 0.75 | 2.25 | 33.33 | 18.00 | 0.05 | 133.33 |
| (A1) Color pigment | | | | | 14.601 | 14.601 | 14.601 | 14.601 | 14.601 | 14.601 | 14.601 |
| (A1)/(A) | | | | | 0.88 | 0.88 | 0.88 | 0.88 | 0.88 | 0.88 | 0.88 |
| (B)/(A) | | | | | 0.181 | 0.181 | 0.060 | 0.181 | 0.001 | 0.422 | 0.181 |
| Evaluation result | | | Base application amount 1 mg/cm² | (1) Finish of base: bright and toned (Evaluation N = 5 out of 5) | 5 | 5 | 4 | 0 | 0 | 2 | 2 |
| | | | With powdery foundation in the same amount | (2) Base + powdery foundation finish: covering roughness and pores (Evaluation N = 5 out of 5) | 5 | 5 | 4 | 2 | 0 | 0 | 0 |
| | | | | (3) Base + powdery foundation finish: smooth finish (Evaluation N = 5 out of 5) | 5 | 5 | 4 | 2 | 0 | 0 | 0 |

**[Table 3]**

| | | | | | Example 11 |
|---|---|---|---|---|---|
| | | | | | Fiber B |
| | | (B) Fiber | | Polymer material | Acrylic resin |
| | | | | Fiber diameter (µm) | 0.6 |
| | | | | Fiber length (µm) | 50 |
| | | | | Aspect ratio | 83.3 |
| | | | | Rate of the number of fibers having a fiber length of 40 µm or more | 54.3 |
| | | | | | Example 11 |
| | | (B) Type of fiber | | | Acrylic resin |
| Powder phase | | (A) Powde r | Color pigment | Silicone-treated yellow iron oxide | 0.01 |
| | | | | Silicone-treated red iron oxide | 0.03 |
| | | | | Silicone-treated black iron oxide | 0.001 |
| | | | | Silicone-treated fine particle zinc oxide (manufactured by TAYCA Corp. MZY-505M) 25 nm | 9 |
| | | | | Silicone-treated fine particle titanium oxide (manufactured by TAYCA Corp. MT-500SA) | 1 |
| | | | | Triethoxycaprylylsilane-treated fine particle titanium oxide (manufactured by Sakai Chemical Industry Co., Ltd., STR-100W) | 4 |
| | | | | Silicone-treated titanium oxide (manufacture by ISHIHARA SANGYOU KAISHA, LTD., CR-50, average particle diameter: 0.25 µm) | 0.56 |
| | | | Extender pigment | Silicone-coated talc (JA-68R) | 2 |
| Oil phase | | (B) Fiber | | Each fiber above | 3 |
| | | (C) Water and nonvolatile oil that is liquid at 20°C | | Dimethylpolysiloxane (KF-96L-6CS) | 4 |
| | | | | Polyisobutene (Parleam EX (pentamer)) | 1 |
| | | | | Isononyl isononanoate | 2 |
| | | | | Diisostearyl malate | 2 |
| Water phase | | | | Purified water | 25 |
| Oil phase | | Surfactant | | Polyether-modified silicone (DOWSIL SH3775M FLUID) | 2.5 |
| | | (D) Volatile oil | | Dimethylpolysiloxane (KF-96L-1.5CS) | 10.699 |
| | | | | Dimethylpolysiloxane (KF-96L-2CS) | 26.5 |
| Water phase | | Other component | | Ethanol | 6.5 |
| | | | | Magnesium sulfate | 0.2 |
| Total amount | | | | | 100 |
| (A) Powder total | | | | | 16.601 |
| (B) Fiber | | | | | 3 |
| (C) Water and nonvolatile oil that is liquid at 20°C | | | | | 34 |
| | Surfactant | | | | 2.5 |
| (D) Volatile oil | | | | | 37.199 |
| (B) (Average fiber diameter)²/fiber content | | | | | 0.12 |
| (A1) Color pigment | | | | | 14.601 |
| (A1)/(A) | | | | | 0.88 |
| (B)/(A) | | | | | 0.181 |

**[Table 4]**

| | | | | | Example 12 |
|---|---|---|---|---|---|
| | | | | | Fiber B |
| | | (B) Fiber | | Polymer material | Acrylic resin |
| | | | | Fiber diameter (µm) | 0.6 |
| | | | | Fiber length (µm) | 50 |
| | | | | Aspect ratio | 83.3 |
| | | | | Rate of the number of fibers having a fiber length of 40 µm or more | 54.3 |
| | | | | | Example 12 |
| | | (B) Type of fiber | | | Acrylic resin |
| Powder phase | | (A) Powder | Color pigment | Silicone-treated yellow iron oxide | 0.01 |
| | | | | Silicone-treated red iron oxide | 0.03 |
| | | | | Silicone-treated black iron oxide | 0.001 |
| | | | | Silicone-treated fine particle zinc oxide (manufactured by TAYCA Corp. MZY-505M) 25 nm | 3 |
| | | | | Silicone-treated fine particle titanium oxide (manufactured by TAYCA Corp. MT-500SA) | 1 |
| | | | | Triethoxycaprylylsilane-treated fine particle titanium oxide (manufactured by Sakai Chemical Industry Co., Ltd., STR-100W) | 4 |
| | | | | Silicone-treated titanium oxide (manufacture by ISHIHARA SANGYOU KAISHA, LTD., CR-50, average particle diameter: 0.25 µm) | 0.56 |
| | | | Extender pigment | Silicone-coated talc (JA-68R) | 8 |
| Oil phase | | (B) Fiber | | Each fiber above | 3 |
| | | (C) Water and nonvolatile oil that is liquid at 20°C | | Dimethylpolysiloxane (KF-96L-6CS) | 6 |
| | | | | Polyisobutene (Parleam EX (pentamer)) | 1 |
| | | | | Isononyl isononanoate | 3 |
| | | | | Diisostearyl malate | 3 |
| Water phase | | | | Purified water | 29 |
| Oil phase | | Surfactant | | Polyether-modified silicone (DOWSIL SH3775M FLUID) | 2.5 |
| | | (D) Volatile oil | | Dimethylpolysiloxane (KF-96L-1.5CS) | 9.699 |
| | | | | Dimethylpolysiloxane (KF-96L-2CS) | 19.5 |
| Water phase | | Other component | | Ethanol | 6.5 |
| | | | | Magnesium sulfate | 0.2 |
| Total amount | | | | | 100 |
| (A) Powder total | | | | | 16.601 |
| (B) Fiber | | | | | 3 |
| (C) Water and nonvolatile oil that is liquid at 20°C | | | | | 42 |
| | Surfactant | | | | 2.5 |
| (D) Volatile oil | | | | | 29.199 |
| (B) (Average fiber diameter)²/fiber content | | | | | 0.12 |
| (A1) Color pigment | | | | | 8.601 |
| (A1)/(A) | | | | | 0.52 |
| (B)/(A) | | | | | 0.181 |

**[Table 5]**

| | | | | Reference prescription |
|---|---|---|---|---|
| Powder phase | (A) Powder | Color pigment | Silicone-treated yellow iron oxide | 0.01 |
| | | | Silicone-treated red iron oxide | 0.03 |
| | | | Silicone-treated black iron oxide | 0.001 |
| | | | Silicone-treated fine particle zinc oxide (manufactured by TAYCA Corp. MZY-505M) 25 nm | 9 |
| | | | Silicone-treated fine particle titanium oxide (manufactured by TAYCA Corp. MT-500SA) | 1 |
| | | | Triethoxycaprylylsilane-treated fine particle titanium oxide (manufactured by Sakai Chemical Industry Co., Ltd., STR-100W) | 4 |
| | | | Silicone-treated titanium oxide (manufacture by ISHIHARA SANGYOU KAISHA, LTD., CR-50, average particle diameter: 0.25 µm) | 0.56 |
| | | Extender pigment | Silicone-coated talc (JA-68R) | 2 |
| Oil phase | (C) Nonvolatile oil that is liquid at 20°C | | Dimethylpolysiloxane KF-96A-1000CS (-G) | 5 |
| | | | Octyl p-methoxycinnamate | 4 |
| | Surfactant | | Polyether-modified silicone (DOWSIL SH3775M FLUID) | 2.5 |
| | (D) Volatile oil | | Dimethylpolysiloxane (KF-96L-1.5CS) | 8.699 |
| | | | Dimethylpolysiloxane (KF-96L-2CS) | 26.5 |
| Water phase | (C) Water | | Purified water | 30 |
| | Other component | | Ethanol | 6.5 |
| | | | Magnesium sulfate | 0.2 |
| Total amount | | | | 100 |

### Reference Signs List

10: electrostatic spraying device
11: syringe
12: high-voltage source
13: conducting collector
11a: cylinder
11b: piston
11c: capillary

## Claims

1. A film-forming composition for skin, comprising components (A) and (B):
(A) a powder; and
(B) a fiber having an average fiber diameter of 0.1 µm or more and 7 µm or less and an aspect ratio (average fiber length/average fiber diameter) of 20 or more and 300 or less in an amount of 0.1 mass% or more and 10 mass% or less relative to the whole film-forming composition, wherein
a mass ratio of the component (B) to the component (A), (B/A), is 0.02 or more and 0.4 or less.

2. The film-forming composition for skin according to claim 1, wherein the component (B) is a fiber of a water-insoluble polymer.

3. The film-forming composition for skin according to claim 1 or 2, wherein the component (B) has an aspect ratio (average fiber length/average fiber diameter) of 25 or more and 300 or less.

4. The film-forming composition for skin according to any one of claims 1 to 3, wherein a content of the component (A) relative to the whole film-forming composition is 0.5 mass% or more and 98 mass% or less.

5. The film-forming composition for skin according to any one of claims 1 to 4, further comprising a component (C) a liquid material selected from the group consisting of water and nonvolatile oils that are liquid at 20°C.

6. The film-forming composition according to any one of claims 1 to 5, wherein the component (A) includes a color pigment.

7. A method for producing a film on a skin surface, comprising applying the film-forming composition according to any one of claims 1 to 6 to the skin.

8. A film comprising the film-forming composition for skin according to any one of claims 1 to 6.

9. Use of the film-forming composition according to any one of claims 1 to 6 as an emulsified cosmetic.

10. Use of the film-forming composition according to any one of claims 1 to 6 for makeup and/or ultraviolet protection by applying the composition to skin, preferably, to face.

11. Use of the film-forming composition for skin according to any one of claims 1 to 6 for producing a powder-containing cosmetic film on a skin surface.
